Europäisches Patentamt

European Patent Office

Office européen des brevets

(19)

(11) Publication number: 0 319 198
A2

# (12) EUROPEAN PATENT APPLICATION

(21) Application number: 88311151.0

(22) Date of filing: 24.11.88

(51) Int. Cl.4: G01N 27/28

(30) Priority: 01.12.87 GB 8728103
15.12.87 GB 8729194

(43) Date of publication of application:
07.06.89 Bulletin 89/23

(84) Designated Contracting States:
AT BE CH DE ES FR GR IT LI LU NL SE

(71) Applicant: UNIVERSITY COLLEGE CARDIFF
CONSULTANTS LTD.
P.O. Box 78
Cardiff, CF1 1XL(GB)

(72) Inventor: Pickard, Robert S.
39 Rannock Drive
Cyncoed Cardiff(GB)
Inventor: Wall, Peter
75 Dunraven Place Wyndham
Ogmore Valley Mid-Glamorgan(GB)

(74) Representative: Cookson, Barbara Elizabeth et
al
WITHERS & ROGERS 4 Dyer's Buildings
Holborn
London EC1N 2JT(GB)

(54) Microenvironmental sensor assemblies.

(57) A sensor array comprises a two dimensional array of sensing interfaces (5) connected by tracks (3) of a deposited metallisation array to connecting pads (2) at which they are monitored electrically. The array may be used for monitoring parameters such as metal ion concentration and pH of in vitro biological material which is cultured over the biocompatible sensing interfaces. Monitoring of the response of cancer cells to cytotoxic drugs is disclosed.

FIG.1.

# MICROENVIRONMENTAL SENSOR ASSEMBLIES

This invention relates to microenvironmental sensors and their applications.

The relatively large size (dimensions greater than $10^{-3}$ - $10^{-7}$m) of many microenvironmental sensors precludes their use for the monitoring of microenvironmental parameters since they modify the environment in which they are placed. Even with microsensors some modification of the environment can occur although the dimensions of such devices reduces this to an absolute minimum.

In one aspect the invention provides a sensor assembly comprising a conductive array deposited on a substrate, said conductive array including a sensor interface or a two-dimensional array of sensor interfaces, exposed on a surface of said assembly, said surface and the exposed portion of the or each said interface being biocompatible and the or each exposed portion having dimensions not exceeding 1mm.

In another aspect the invention provides a method of sensing the state of in vitro biological material comprising culturing said material on a biocompatible surface carrying exposed biocompatible sensing interface means and sensing the electrical state of said interface means. This allows the biological material to be monitored.

The sensors may have any required shape, for example, a square, rectangular or circular substrate carrying a single sensing interface or a predetermined array of such interfaces. Each interface is preferably of micro dimensions (less than 10 microns) and the position of the interface may be customised and specific for each application. Signal processing circuitry is required to collect and interpret the data from each interface and each sensor is used with appropriate circuitry.

By using standard semiconductor fabrication technologies and materials, microenvironmental transducers of micro dimensions may be produced that do not need to be introduced into the microenvironment to monitor it. Cells may grow over the array and create their microenvironment around the sensors, so the array is free from the disadvantages that always accompany the introduction of sensors.

The sensor array, which may consist of one or more sensing interfaces, is suitably made on a glass, polymer, plastics or silicon substrate. In addition the substrate may be rigid or flexible. The spatial geometry of the sensing interfaces can also be modified and specified for precise applications or requirements.

The sensor array is suitably defined using standard semiconductor technology, such as photolithography, or electron beam lithography, which enables great precision and repeatability in sensor interface dimensions and spatial location within the sensor array. The sensors of the invention may be designed to be used in conjunction with signal processing circuits and to be readily interfaced with such instrumentation by the use of standard interfacing methods and materials. This enables sensors of differing sensory modalities to be readily and quickly interchanged with the signal processing circuitry.

The sensors of the invention are primarily designed for use in culture dishes to monitor the growth and activity and microenvironments of living cells. The effects of added agents can be determined and the devices thus provide in addition a rapid and efficient method of screening and evaluating the effects of materials, e.g. cytotoxic drugs, on the living cells. In this way the effects of modifications to the microenvironment of the cells can also be monitored. In the case of tumour cells the monitoring of the oxygen concentrations and the activity of other free radical stoppers within the cells and their microenvironment can be used to determine the optimum time to irradiate the cancerous cells.

A sensor array in accordance with the invention may be incorporated into the bottom of a culture vessel either as a separate substrate or as an integral part of the culture vessel and cells cultured on top of the sensing array in appropriate culture media. In this way the microenvironment of the material in the culture vessel is not disturbed by the sensors.

The sensing interface form and function (modality) can be selected prior to use thus enabling many parameters within the microenvironment to be determined at the same time, non-invasively.

The fabrication of use of a sensor in the monitoring of human cancer cells growing in vitro, is described below, by way of example only, with reference to the accompanying drawings, in which:

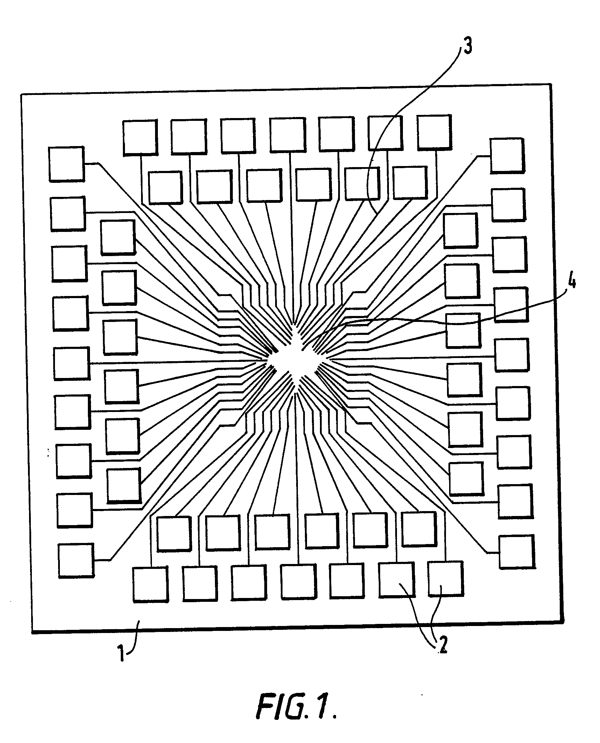

Figure 1 is a plan view of a multisite sensor array; and

Figure 2 is a magnified surface view of the central portion of the sensor showing multisensing interfaces.

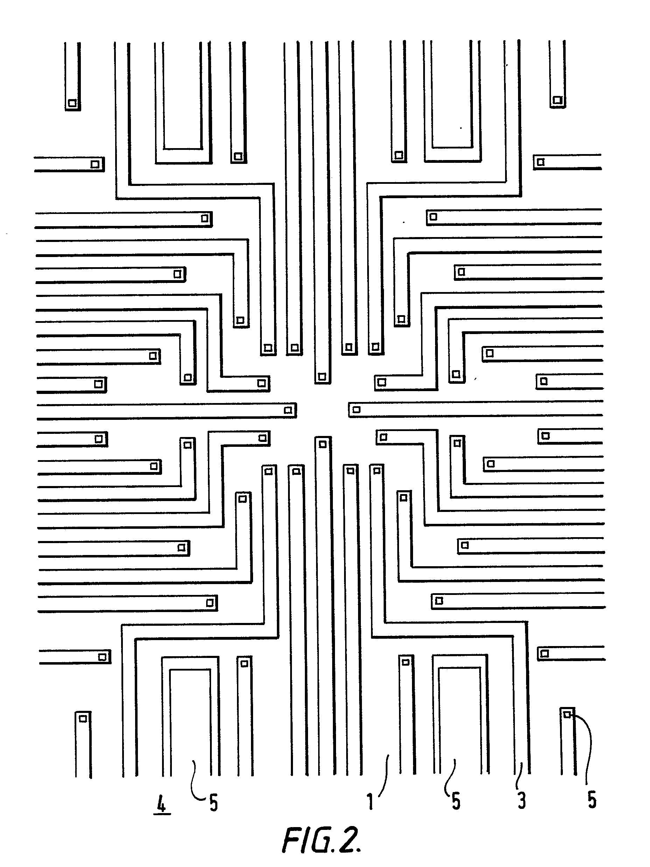

The sensor consists of a polymer, glass, plastics or silicon substrate 1 forming a square body of dimensions 14000 microns x 14000 microns. The substrate may be 900 microns thick. The sensor array 4 illustrated has 56 sensing sites arranged in a two-dimensional array. Each site contains one sensor interface 5 and any subset of these can be interrogated simultaneously from metallic connection pads 2 which are linked by metallic tracks 3 to the sensing interfaces 5 (Figure 2) which may be arranged to sense for example, pH, oxygen, temperature and other ions within or beneath cultured cells and tissues.

The arrangement of the conductors is illustrated in Figure 2, in which each sensing interface is connected by a separate and isolated conductor 3 to a respective pad 2.

The materials used for the sensor interface and insulators must be biocompatible. Preferred metals are tantalum, titanium, gold, platinum, iridium and rhodium.

The insulation material may comprise for example silicon dioxide and/or silicon nitride or biocompatible polymer.

The array can either be incorporated into a culture vessel (not shown) as an individual array or integrated into/onto the fabric of the culture vessel.

Other applications include monitoring the effects of materials added to the culture vessel and its contents. For example, in the case of in vitro growth of human cancer cells, the effects of the addition of cytotoxic drugs, chemicals and therapeutic agents on the cultured material can be monitored.

The sensing interface array pattern can be customised for specific applications and different chemical species that are to be detected.

In the described embodiment, the dimensions of the exposed portions of the sensing interfaces do not exceed $10^{-2}$mm. They are typically 2 - 6 microns and most preferably 4 microns. However, in some cases the exposed portions of the sensing interfaces may have one or more dimensions exceeding this.

## Claims

1. A sensor assembly comprising a conductive array deposited on a substrate (1), said conductive array including a sensor interface (5) or a two-dimensional array (4) of sensor interfaces (5), exposed on a surface of said assembly, said surface and the exposed portion of the or each said interface (5) being biocompatible and the or each exposed portion having dimensions not exceeding 1mm.

2. A sensor assembly as claimed in claim 1, wherein the conductive array defines a respective metallisation or other suitable conducting material track (3) for each interface (5), each said track (3) terminating in a connection pad (2).

3. A sensor assembly as claimed in claim 1 or claim 2, wherein the or at least one said sensing interface (5) is adapted to measure temperature, pH, oxygen concentration or cation concentration.

4. A sensor assembly as claimed in any one of the preceding claims, wherein said surface is an interior surface of a culture vessel.

5. A sensor assembly as claimed in any one of the preceding claims, wherein said dimensions do not exceed $10^{-2}$mm.

6. A sensor assembly as claimed in any one of the preceding claims, wherein the dimensions are selected not to exceed those of cells to which the surface is to be exposed.

7. A method of sensing the state of in vitro biological material comprising culturing said material on a biocompatible surface carrying exposed biocompatible sensing interface means, and sensing the electrical state of said interface means.

8. A method as claimed in claim 7, comprising the further step of adding a cytotoxic drug to said material.

9. A method as claimed in claim 7 or 8, carried out using a sensor assembly as claimed in any one of claims 1 to 6.

FIG.1.

*FIG.2.*